# EUROPEAN PATENT APPLICATION

(11) **EP 2 719 379 A1**
(43) Date of publication of application: **16.04.2014**
(21) Application number: 13197507.0
(22) Date of filing: 17.04.2009
(51) Int. Cl.: A61K 31/05, A61K 36/63, A61P 1/16, A61P 7/00, A61P 9/00, A61P 21/00, A23L 1/00

(54) **Hydroxytyrosol benefits mitochondria**

(30) Priority: 17.04.2008 EP 08007492
(62) Divisional of application: 09753748.4
(71) Applicant: DSM IP Assets B.V., 6411 TE Heerlen (NL)
(72) Inventor: Liu, Jiankang, 4303 Kaiseraugst (CH); Raederstorff, Daniel, 4303 Kaiseraugst (CH); Wang-Schmidt, Ying, 4303 Kaiseraugst (CH); Wertz, Karin, 4303 Kaiseraugst (CH)
(74) Representative: Dux, Roland

(57) **Abstract**

Hydroxytyrosol or olive juice containing hydroxytyrosol can be used to maintain or increase mitochondrial biogenesis in cardiac muscle, skeletal muscles, and liver tissue.

## Description

### FIELD OF THE INVENTION

This invention is related to the use of hydroxytyrosol ("HT"), or an olive juice extract containing hydroxytyrosol as an agent to improve or maintain mitochondrial health or as a mitochondrial biogenesis agent. It also relates to pharmaceutical and nutraceutical compositions useful for conditions characterized by altered mitochondrial functioning and biogenesis, such as heart strength, various liver diseases, improve muscle /fat ratio and muscle endurance.

### BACKGROUND OF THE INVENTION

Mitochondria are organelles in the cell responsible for aerobic energy production. The mitochondrial inner membrane is embedded with a respiratory chain containing complexes I, II, III, IV and V, which transport electrons and produce ATP via a series of redox reactions, a process called oxidative phosphorylation. The aerobic energy metabolism is more efficient than the anaerobic energy production. Anaerobic energy production involves the conversion of glucose to lactate (glycolysis), and generates only 8 Mol ATP per Mol glucose. During aerobic energy metabolism, glucose is completely oxidized (by glycolysis, Krebs cycle and the mitochondrial electron chain) to CO2 and H2O, while giving rise to 38 Mol ATP/mol glucose.

There is a critical metabolic fork in the road at the end of glycolysis. At this fork, glucose has been converted from one 6 carbon molecule to two, 3 carbon molecules called pyruvic acid, or pyruvate. This pyruvate can either be shuttled into the mitochondria via the enzyme pyruvate dehydrogenase, or converted to lactic acid via the enzyme lactate dehydrogenase. Entry into the mitochondria exposes the pyruvate to further enzymatic breakdown, oxidation, and a high ATP yield per glucose. This process inside the mitochondria ultimately requires oxygen molecules to proceed and is therefore "aerobic". Conversion to lactate means a temporary dead end in the energy yielding process, and the potential for contractile fatigue due to decreasing cellular pH if lactic acid accumulation proceeds unchecked.

In addition to their well known function of supplying energy to a cell, mitochondria and their components participate in a number of other cellular activities. For example, mitochondria also control thermogenesis and the apoptosis process and are thus involved in the ageing process.

The mitochondria contain a high level of oxidants, since the respiratory chain generates reactive species, e.g. superoxide anions, if it works with reduced efficiency or during energy uncoupling. Superoxide anions are generated as byproducts in several steps of electron transport chain, such as the reduction of coenzyme Q in complex III, where a highly reactive free radical is formed as an intermediate (Q·-). This unstable intermediate can lead to electron "leakage", when electrons jump directly to oxygen and form the superoxide anion, instead of moving through the normal series of well-controlled reactions of the electron transport chain.

An antioxidant is a molecule capable of slowing or preventing the oxidation of other molecules. Antioxidants terminate oxidation chain reactions by removing free radical intermediates, and inhibit other oxidation reactions by being oxidized themselves. Reducing agents such as thiols or polyphenols often exert antioxidant property. Well known antioxidants such as Vitamin A, C and E scavenge free radicals and protect DNA, proteins and lipids from damage. Antioxidants also protect mitochondria from reactive oxygen species and free radicals generated during ATP production.

While it has been generally accepted in the past that administration of antioxidants would be beneficial to promote mitochondrial biogenesis, this has not been shown to be the case. Gomez-Carbera et al. 2008 Am. J Clin. Nutr. 87(1):142-149, demonstrated in a double-blinded randomized clinical study, that oral administration of 1g Vitamin C per day actually resulted in decreased mitochondrial biogenesis in skeletal muscle.

Hydroxytyrosol has been described in the past as having positive cardiovascular effects (see, e.g. Gonzalez-Santiago et al 2006 Atherosclerosis 188:35-42; or Mitro et al 2003 NMCD. Nutritional Metabolism and Cardiovascular Diseases 13(5):306; but these are concerned with the anti-atherosclerotic effects of hydroxytyrosol and/or its status as an antioxidant.

### DETAILED DESCRIPTION OF THE INVENTION

It has been found, in accordance with this invention, that hydroxytyrosol ("HT") induces mitochondrial biogenesis and can lead to an increased mitochondrial function in tissues. Thus one aspect of this invention is a method of maintaining or increasing mitochondrial function and activity via increased mitochondrial biogenesis comprising administering an effective amount of hydroxytyrosol to a mammal.

Mitochondrial biogenesis refers to processes of growth, amplification and healthy maintenance of the mitochondria. Mitochondrial biogenesis is a complex process involving both nuclear and mitochondrial players. Mitochondrial DNA encodes a small number of proteins, which are translated on mitochondrial ribosomes. Most of these proteins are highly hydrophobic subunits of the respiratory chain, which is localized in the inner mitochondrial membrane. Nuclear-encoded proteins are translated on cytosolic ribosomes and imported into mitochondria. These proteins include structural proteins, enzymes or enzyme subunits, components of the import-, replication-, transcription- and translation-machinery and chaperones.

Cells have to switch to the less efficient anaerobic energy metabolism, once the capacity for the aerobic respiration (electron chain) does not suffice anymore. It follows that increased mitochondrial biogenesis improves the capacity for aerobic energy metabolism, and thus increases the capacity for an efficient energy production.

"Mitochondrial biogenesis" as used throughout this specification and claims, includes all processes involved in maintenance and growth of the mitochondria, including those required for mitochondrial division and segregation during the cell cycle.

As used throughout the specification and claims, the term "biogenesis-inducing amount" means that the overall mitochondrial biogenesis is at least maintained at the level which was present when the hydroxytyrosol was originally ingested. This can be determined *in vitro* by monitoring the amount and state of mitochondrial functioning in a tissue sample, or as described further in the Examples. Additionally, this can be determined *in vivo* by measuring the ATP content of tissue; or the oxygen consumption during exercise (VO₂ max), or *ex vivo* by transcriptomics analysis for upregulation of mitochondrial markers (such as Tfam), or by detecting the increased presence of mitochondrial DNA in tissue biopsies. This property of hydroxytyrosol is distinct from hydroxytyrosol's known activity as an antioxidant.

"Mitochondrial-stimulating" as used throughout this specification and claims means that the compound applied to the mitochondria leads to increased ATP production in the cell; an increased capacity for energy production in the cell; an increased capacity for aerobic energy generation or production in the cell; and/or an increased capacity for fat burning.

Hydroxytyrosol (3,4-dihydroxyphenylethanol) may be of synthetic origin or it may be isolated from extracts of olive leaves, olive fruits, olive pulp, or vegetation water of olive oil production. Thus, the term "hydroxytyrosol" also encompasses any material or extract of a plant or any material or extract of parts of a plant or any extract/concentrate/juice of fruits of a plant (such as olives) containing it, especially in an amount of at least 1.5 weight %, preferably in an amount of at least 30 weight %, and more preferably in an amount of at least 40 weight-%, more preferably in an amount of at least 50, 55, 60, 65, 70, 75, 80, 85, 90 weight-%, and most preferably in an amount of at least 45 weight-%, based on the total weight of the plant material or extract. The commercial form of the extract may or may not be standardized to lower concentrations of hydroxytyrosol by formulating the hydroxytyrosol with suitable formulation exicipients. The terms "material of a plant" and "plant material" used in the context of the present invention means any part of a plant, also the fruits.

In further embodiments of the present invention, hydroxytyrosol derivatives such as esters and physiologically/pharmaceutically acceptable salts may be used instead of or in addition to hydroxytyrosol. It is also possible to use a mixture of hydroxytyrosol and hydroxytyrosol derivatives. Derivatives can be e.g. esters or glucosides, and are known to the person skilled in the art. Preferred esters of hydroxytyrosol are e.g. acetates or glucuronide conjugates; as well as oleuropein being the most preferred one.

Thus, one aspect of this invention is the use of hydroxytyrosol in the manufacture of a medicament or food product (for humans and/or animals) which is useful for maintaining or increasing mitochondrial biogenesis or mitochondrial function. Another aspect of this invention is a method of maintaining or increasing mitochondrial biogenesis in a subject in need thereof comprising administering a mitochondrial biogenesis-inducing or mitochondrial-stimulating amount of hydroxytyrosol.

Another aspect of this invention is the use of hydroxytyrosol in the manufacture of a medicament or food product (for humans and/or for animals) which is useful in protecting mitochondria against any number of stresses found in the daily environment. These products help to ensure normal mitochondrial function in the face of everyday insults, such as cellular biochemical changes as a result of stresses, illnesses, malnutrition (including malnutrition which is secondary to another disease state) or injury.

Another aspect of this invention are nutraceuticals which comprise a mitochondrial biogenesis-inducing amount of hydroxytyrosol, and which promote mitochondria well-being and encourage optimal mitochondrial function.

Another aspect of this invention is a cosmetic composition which comprises a mitochondrial biogenesis-inducing amount of hydroxytyrosol, and which promotes mitochondria well-being and which encourages optimal mitochondrial function in the skin, and thus which boosts the energy metabolism of the skin.

### BRIEF DESCRIPTION OF THE FIGURES

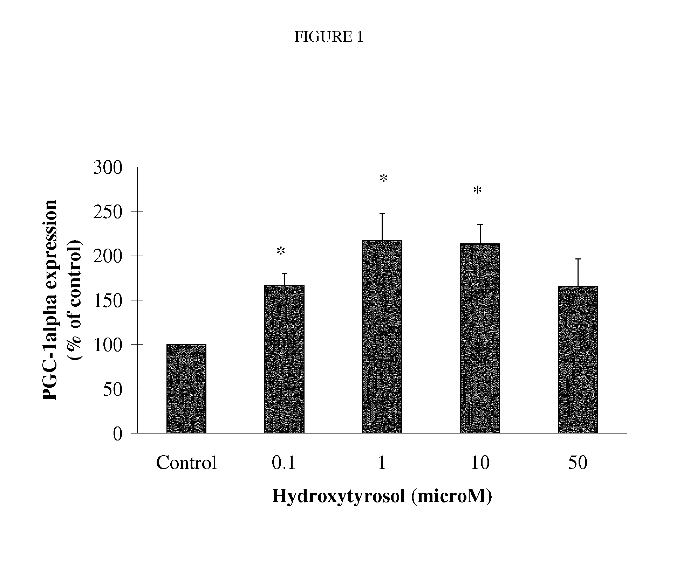

FIGURE 1 shows expression of PGC-1α. Quantitative values tabulated for *PGC-1α:* α-tubulin ratio with a densitometry. Values are mean ± SE of five experiments. * p< 0.05 vs. control; **P<0.01 vs.control.

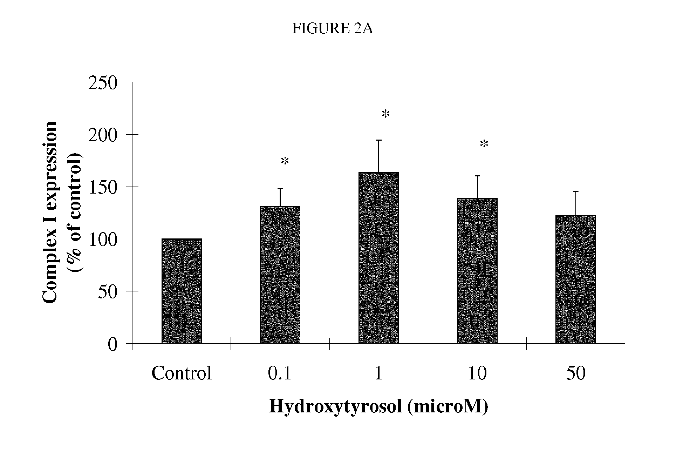

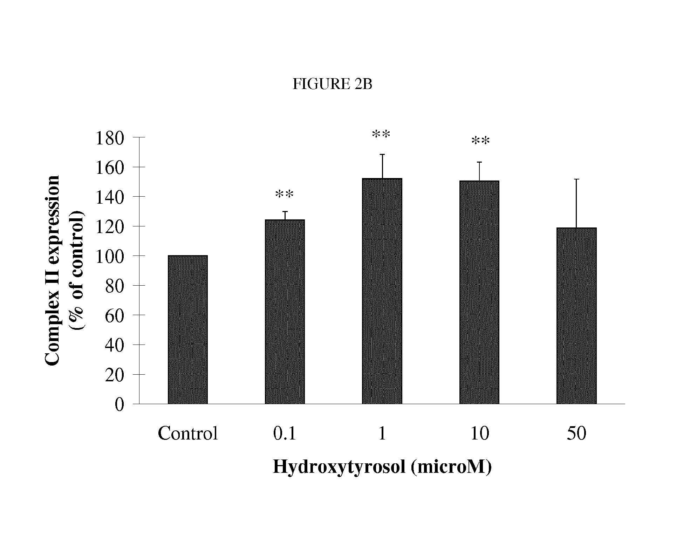

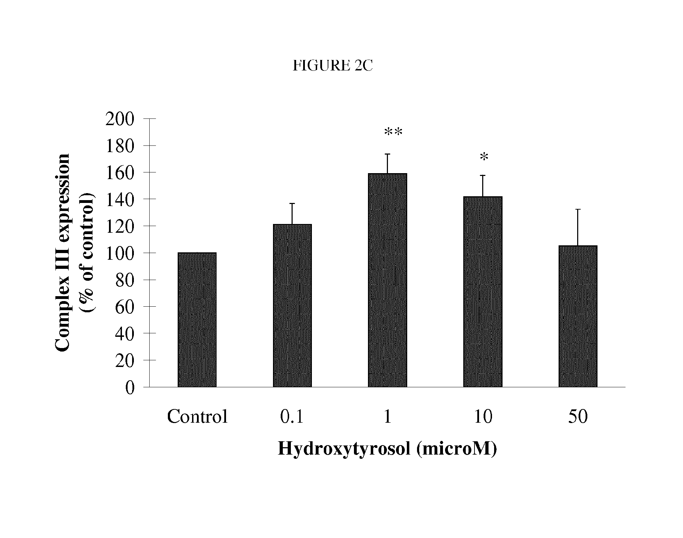

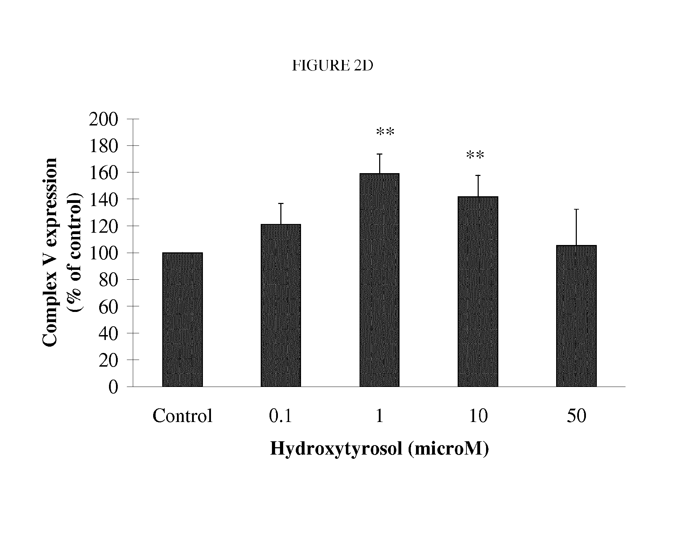

FIGURE 2 shows expression of mitochondrial proteins. 3T3-L1 adipocytes were treated for 48 hrs with hydroxytyrosol. Cells were subsequently solubilized into SDS sample buffer and analyzed by Western blotting with antibodies against α-tubulin, mitochondrial electron transport complexes. The quantitative analyses of the bands by densitometry are shown in A, B, C and D for mitochondrial complex I, complex II, complex III and complex V, respectively. Results shown are fold increases from control from 4 independent experiments compared with control cells. *p < 0.05 vs. control. **p<0.01 vs.control.

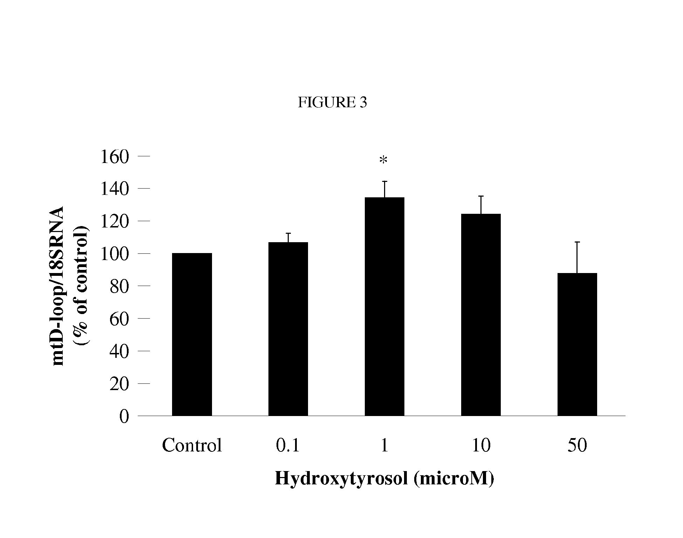

FIGURE 3 shows expression of mitochondrial DNA. 3T3-L1 adipocytes were treated for 48 hrs with hydroxytyrosol. PCR products were quantified by fluorescence using SYBR Green. Quantitative values tabulated for D-loop: 18sRNA ratio. Results are expressed as % of control. Data are mean±SE (n=5). *P<0.05 vs. control; ** p<0.01 vs. control.

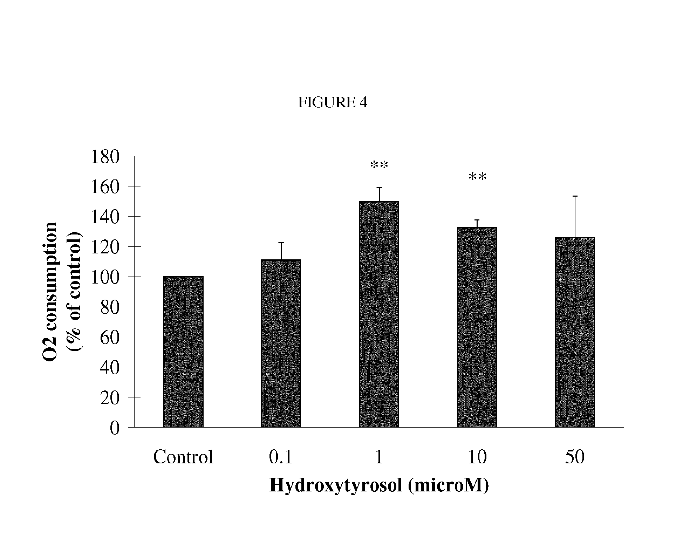

FIGURE 4 shows oxygen consumption in 3T3-L1 adipocytes. Equal volumes of cells were separated into aliquots in wells of a 96-well BD Oxygen Biosensor plate. Plates were covered and fluorescence in each well was recorded over time with a fluorescence microplate spectrophotometer. Quantitative changes in the respiratory rate of adipocytes during each condition were calculated by determining the kinetic measurements. Values are mean ± SE; results shown are % of control from 3 independent experiments compared with control cells. *p< 0.05 vs. control.

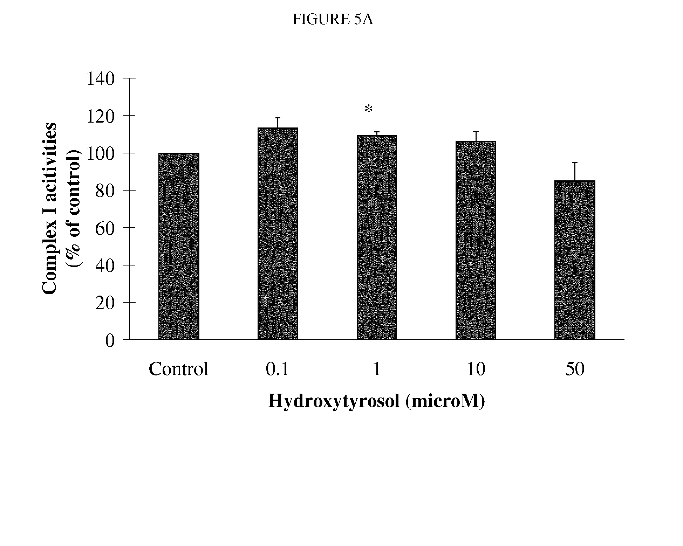

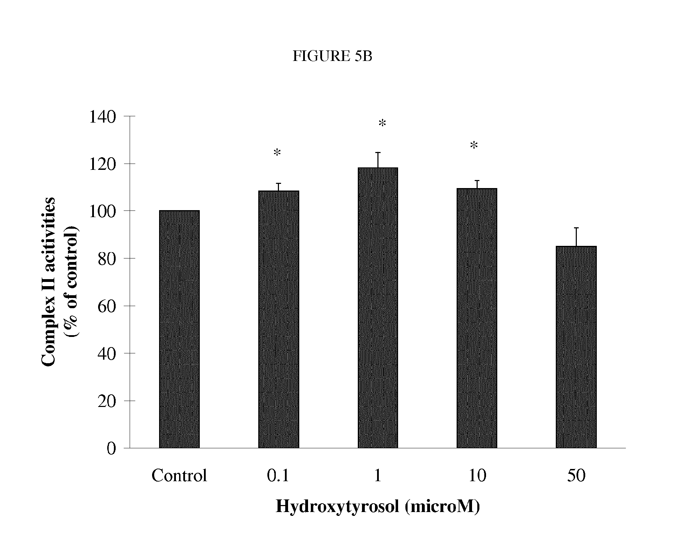

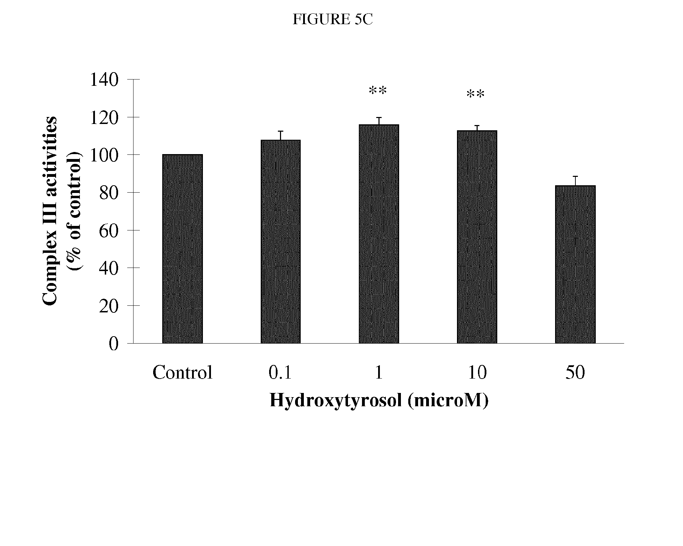

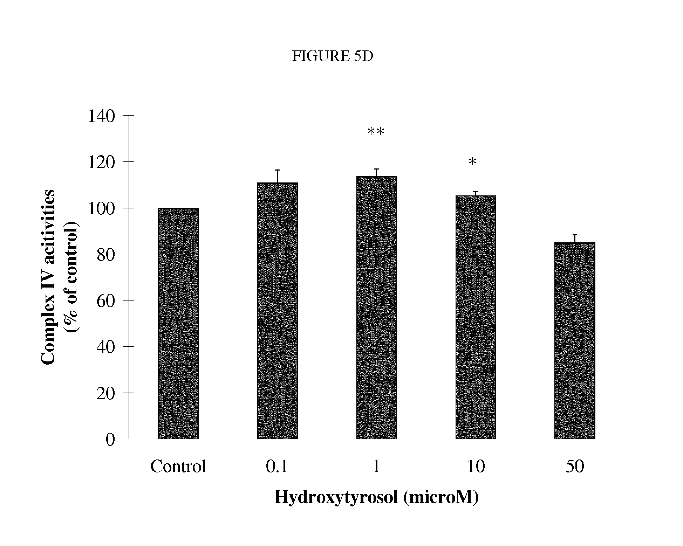

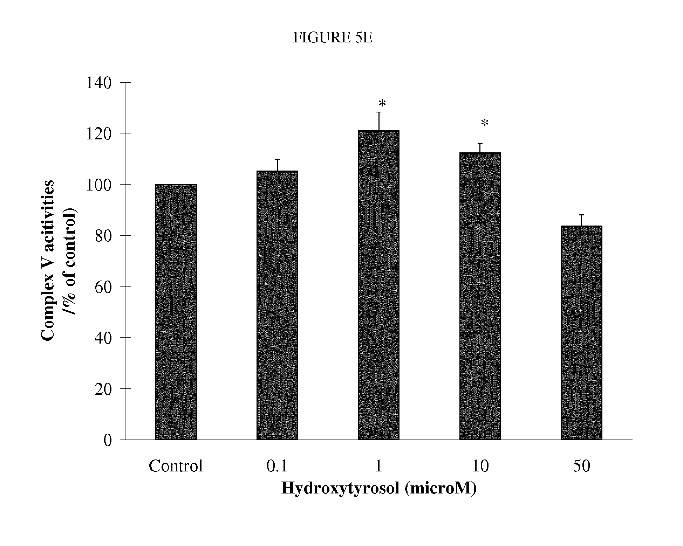

FIGURE 5 shows the effect of treatment with hydroxytyrosol on activities of complexes in adipocytes. (A) Complex I, (B) Complex II, and (C) Complex III, (D) Complex IV and (E) Complex V Adipocytes were treated with different concentrations of hydroxytyrosol for 48 hrs. Values are mean ± SE of data from three separate experiments for complex I, and six separate experiments for complex II and III, and each experiment was performed in duplicate.*p<0.05, **p <0.01 vs. control.

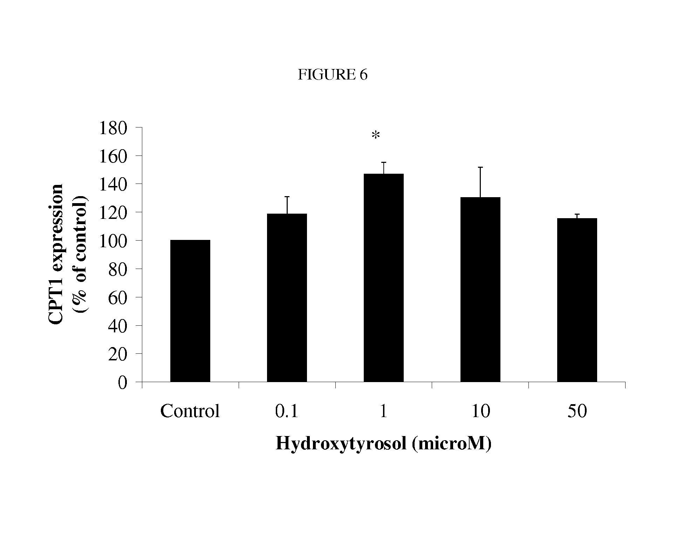

FIGURE 6 shows the effect of HT treatments on expression of Cpt1a mRNA. 3T3-L1. Adipocytes were treated for 48 hrs with HT at 0.1, 1.0, 10, and 50 µmol/1, and total RNA was isolated. The cycle number at which the various transcripts were detectable was compared with that of 18S rRNA as an internal control. Results are expressed as % of control. Values are mean ± SE of the results from at least four independent experiments. *P<0.05 vs. control without HT treatment.

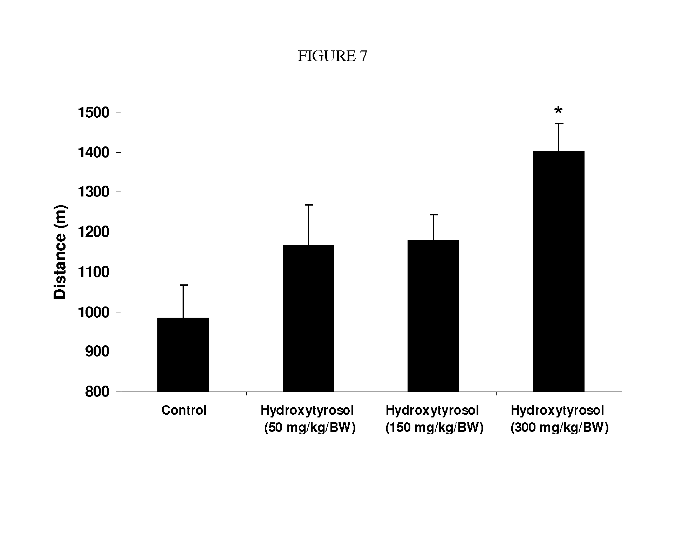

FIGURE 7 shows hydroxytyrosol, given as an olive extract containing 50% hydroxytyrosol at doses of 50, 150, or 300 mg/kg body weight per day by gavage, increases endurance by up to 50% in mice after 3 weeks of supplementation.

PGC1α, Peroxisome proliferation activator receptor (PPAR) gamma-coactivator 1 alpha, a transcription coactivator, functions as a master regulator of a wide array of metabolic and physiological processes and is an essential factor in the process of mitochondrial biogenesis. PGC-1α overexpression stimulates mitochondrial biogenesis in 3T3 cells making them more resistant to oxidative stressors.

The inventors have demonstrated that hydroxytyrosol at 1.0-10µM, increases PGC1 α protein level and promotes mitochondrial biogenesis. Thus, nutritional supplementation of hydroxytyrosol will increase mitochondrial activity and prevent mitochondrial dysfunction in different tissues. Further, hydroxytyrosol can thus maintain tissue/organ function and prevent tissue/organ failure triggered by mitochondrial dysfunction.

### Conditions which can benefit from maintaining or increasing mitochondrial biogenesis

### A. Cardiac Health and Strength

An increased mitochondrial biogenesis results in a number of desirable physical states. One example would be an increase in cardiac health, energy, strength and endurance. The heart, with an increased biogenesis of mitochondria has access to a greater store of energy. A heart muscle which has access to more energy will have stronger contractions, and will provide better blood circulation. Therefore, people with a heart disease, injury, or a condition which has resulted in a weak heartbeat (such as those who have had scarlet fever) will benefit from increasing cardiac muscle mitochondrial biogenesis. Also those with problems connected with poor circulation (such as those with diabetes, or people with cold hands or feet) will also benefit from a stronger heart muscle which can provide for better blood circulation. Additionally, people concerned with maintaining their current healthy heart or preventing heart disease which results in weak contractions by the heart will benefit from increasing the mitochondrial biogenesis of the cardiac muscle. Hydroxytyrosol's mitochondrial biogenesis which benefits heart health and as used in this specification and claims is to be considered distinct from heart benefits such as prevention of atherosclerosis, and other vascular-related heart health which are known in the literature. Here, the heart muscles themselves are strengthened and benefitted rather than the blood vessels contributing to circulation.

### B. Liver function

The liver plays a key role in the body's metabolism, especially in energy metabolism, nitrogen excretion and the elimination of toxins from the body. It acts as a filter to remove toxins and waste products from the body, stores nutrients and plays a role in managing levels of certain chemicals in the body, such as cholesterol, hormones, and sugars. The liver can be damaged by various factors such as excessive alcohol intake, cancer, genetic liver disorders, or infections such as hepatitis B or C. Moreover, fatty livers diseases lead to liver dysfunction, in particular nonalcoholic fatty liver disease is a cause of liver-related morbidity and mortality.

Fatty liver diseases cover different conditions including alcoholic liver disease (ALD) and non-alcoholic fatty liver disease (NAFLD). Fatty liver diseases may develop due to various triggers such as nutrition (e.g. protein-calorie malnutrition, starvation, total parenteral nutrition, rapid weight loss, gastrointestinal surgery for obesity), drug use (e.g. glucocorticoids, synthetic estrogens, aspirin, calcium-channel blockers, tetracycline), alcohol use, metabolic or genetic disorders (e.g. lipodystrophy, dysbetalipoproteinemia, Weber-Christian disease, galactosaemia, glycogen storage disorders, acute fatty liver of pregnancy), viral infection (Hepatitis B or C), bacterial infections, obesity, and/or hyperlipidaemia Alcoholic liver disease (ALD) is due to heavy alcohol consumption and is a major cause of cirrhosis. The condition ranges from steatosis through steatohepatitis, cholestasis, fibrosis (liver scarring) and ultimately cirrhosis (advanced irreversible scarring of the liver) and liver failure.

Non-alcoholic fatty liver disease (NAFLD) refers to a spectrum of conditions ranging from simple steatosis which progress to nonalcoholic steatohepatitis (NASH), cholestasis, advanced fibrosis and cirrhosis. The pathological picture resembles that of alcohol-induced liver injury, but it occurs in patients who do not abuse alcohol and is due to other triggers or liver disturbance. Steatosis is characterized by the accumulation of triacylglycerol in hepatocytes. Non-alcoholic steatohepatitis (NASH) is a later stage within the spectrum of NAFLD where steatosis is associated the development of necroinflammation and fibrosis (liver scarring) which can progress to cirrhosis (advanced irreversible scarring of the liver) and liver failure or hepatocellular carcinoma.

Non-alcoholic fatty liver disease (NAFLD) is a common condition with a prevalence higher than 20% in adults aged 45-55 in Western countries. The risk of NASH is increased with obesity and in subjects with high blood lipid levels. However, some patients with NASH are not obese and have normal blood cholesterol and lipids; therefore, some forms of NASH are not simply due to obesity that affects the liver. Fatty liver diseases are very common and there is lack of effect treatment and a high interest in new treatments.

Recently, progress in the physiopathologic mechanisms showed that mitochondrial dysfunction is the main mechanism implied in the progression of fatty acid liver diseases. Mitochondria are organelles generating energy in cells by converting nutrients in adenosine triphosphate (ATP), those molecules are used for normal cell functioning and maintenance. Thus, mitochondria play a major role in fat oxidation and energy production and mitochondrial dysfunction lead to dysfunctional cell and organs in the body. In particular, mitochondrial dysfunction has been shown to play a key role in the progression of fatty liver diseases.

A mouse model with a heterozygous defect of a mitochondrial trifunctional protein which catalyzes long-chain fatty acid oxidation showed an accelerated and increased steatosis. Mitochondrial lesions are also observed in other rodent models that develop hepatic steatosis.

Patients with NAFLD have ultrastructural lesions in their mitochondria and defective mitochondrial functions. Electronic microscopy revealed that mitochondria in NAFLD patients are enlarged, swollen and their number is decreased. Moreover, these mitochondria have decreased expression of mtDNA-encoded polypeptides and markedly decreased activities of respiratory chain complexes (complex I, II, IV and V). Mitochondrial respiration is also significantly decreased in chronic hepatitis and more in liver cirrhosis. The hepatic mitochondria of patients with NASH have a decreased ATP re-synthesis rate after a fructose challenge, which transiently depletes hepatic ATP. Thus patients with NAFLD are unable to maintain cellular ATP levels due to mitochondrial dysfunction which leads to fatty liver diseases. The data suggest that increasing mitochondrial activity, particularly mitochondrial respiratory activity and fatty acid beta oxidation capacity, can be helpful in the management of fatty liver diseases.

Hydroxytrosol promotes mitochondrial function and as such can be used to prevent, treat or alleviate fatty acid liver diseases particularly non-alcoholic fatty liver diseases (NAFLD), steatosis and steatohepatitis lesions. In general, hydroxytyrosol can thus be used to support liver health, to assist the liver in maintaining healthy liver fat metabolism, and to maintain a healthy liver metabolism overall.

### C. Muscle function

In addition to providing benefits to the heart muscle, hydroxytyrosol can benefit skeletal muscles by inducing or maintaining mitochondrial biogenesis.

An increase in mitochondria can benefit both people and animals involved in exercise. Mitochondria biogenesis translates into increase oxygen usage and increased energy while engaging in any form of exercise. Higher mitochondrial volumes improve the capacity for oxidative metabolism at high glycolytic flux rates. Further, this results in increased endurance. The muscle fibers, which have access to greater energy stores are able to contract faster and more fully; thus improvements in speed and strength can be seen after usages over a period of time.

Additionally, improved fatty acid oxidation capacity results in decreased glucose utilization at submaximal exercise intensities. Moreover, fat metabolism proceeds via a different pathway than glucose, and lactic acid is not produced. Also, recovery times from injury, cramps, and soreness resulting from anaerobic energy production will be quicker.

In another muscular application, muscles which utilize energy more efficiently are less prone to fat buildup, and therefore, hydroxytyrosol can be used to improve body-shaping.

Veterinary applications of the mitochondrial biogenesis inducing amout of hydroxytyrosol include: to increase performance in race animals, such as race horses and dogs, and racing camels; to increase endurance in draft animals.

### D. Feeling energetic

In addition to improving physical performance in exercising people, HT can help to generate the energy needed for today's lifestyles. Consumer research showed that 37% of respondents (general population, Age 16+, US and 4 European countries) often felt tired or lacked energy. A second study found that 60% of respondents would be interested in products helping to have more energy. Today, people in need of an energy boost, consume coffee or caffeine-containing energy beverages, as well as high sugar, high fat snacks.

If consumed at the right dose, the immediate effect of caffeine to relieve tiredness is much appreciated. However, if overconsumed, it can cause sleeplessness and hypertension. A typical cup of coffee can contain approximately 100 mg caffeine. If a person regularly drinks 3-5 cups per day, the caffeine consumption can be quite high (+300 mg per day). Hydroxytyrosol, due to its stimulating effect on mitochondrial biogenesis, can add a long term effect of sustained energy to the short term immediate effect of caffeine.

Moreover, hydroxytyrosol can over time help reduce the caffeine dose needed to cope with the day's duties. With the addition of hydroxytyrosol to the diet, the same non-lethargic feeling can be achieved, but the amount of caffeine needed is reduced (to 100 mg or less per day).

Similarly, consumers often eat foods high in sugars and/or fat to provide an energy boost. However, with the sustained energy increase from hydroxytyrosol, the amount required is reduced. Thus, hydroxytyrosol can favourably be used in "better for you" (BFY) energy offers (drinks, bars, snacks, gums, shots, supplements and the like) with a lower content in calories from sugar or fat, or in caffeine.

Thus present invention is directed to the use of hydroxytyrosol for
- increasing body's own capacity for energy generation
- increasing the aerobic capacity for exercise
- shifting nutrient usage for energy generation from carbohydrate to fat burning
- complementing the immediate short term effect of caffeine with a sustained effect on energy generation
- allowing one to reduce the caffeine and /or sugar and/or fat dose needed to "keep going".

Thus, another aspect of this invention is a composition comprising hydroxytyrosol to maintain or increase mitochondrial biogenesis, wherein the maintained or increased mitochondrial biogenesis results in a sustained energy boost. A further aspect of this invention is a method of making a food composition which provides energy or alertness enhancement comprising adding hydroxytyrosol. Yet another aspect of this invention is a method of making a food composition which provides energy or alertness enhancement comprising hydroxytyrosol wherein the food composition contains a reduced caffeine, fat, and/or sugar content compared to a similar food item which does not contain hydroxytyrosol.

### E: improve muscle/fat ratio, body shaping

The present invention is directed to the use of hydroxytyrosol for
- increasing muscle metabolism to boost energy mobilization;
- improving skeletal muscle mass by stimulating anabolic pathways, inhibiting catabolic pathways and accelerating muscle regeneration when damaged;
- shifting nutrient usage for energy generation from carbohydrate or protein burning to fat burning;
- promoting fat burning; acting as a regulator of fat burning, increasing energy expenditure by fatty acid oxidation, increasing fat metabolism, promoting fat oxidation, decreasing body fat and increasing muscle mass;
- helping to achieve a good silhouette (body shaping), decreasing body fat and increasing lean muscle mass; and
- increasing thermogenesis; increasing the metabolism of a human or animal to burn more energy;

### Formulations

Hydroxytyrosol or olive juice extracts containing hydroxytyrosol according to the present invention can be used in any suitable form such as a food, or a beverage, as Food for Special Nutritional Uses, as a dietary supplement, as a nutraceutical or in animal feed or food.

The hydroxytyrosol or olive juice extracts containing hydroxytyrosol may be added at any stage during the normal process of these products. Suitable food products include e.g. cereal bars, bakery items such as cakes and cookies or other types of snacks such as chocolate, nuts, gummy bears, chewing gums, and the like, and also liquid foods such as soups or soup powders. Suitable beverages encompass non-alcoholic and alcoholic drinks as well as liquid preparations to be added to drinking water and liquid food. Non-alcoholic drinks are preferably mineral water, sport drinks, energy drinks including those containing glucuronolactone for increased mental alertness and taurine for detoxification, hybrid energy drinks, near water drinks, fruit juices, lemonades, smoothies, teas and concentrated drinks such as shots and mini-shots. The sports drinks can be hypotonic, hypertonic or isotonic. Sports drinks can be available in liquid form, as concentrates or as powder (to be dissolved in a liquid, as for example water). Examples of Foods for Special Nutritional Uses include the categories of sport food, slimming foods, infant formula and clinical foods. Feed includes any animal food or feed premix, including items such as pet treats and snacks.

The term "dietary supplement" as used herein denotes a product taken by mouth that contains a compound or mixture of compounds intended to supplement the diet. The compound or mixture of compounds in these products may include: vitamins, minerals, herbs or other botanicals and amino acids. Dietary supplements can also be extracts or concentrates, and may be found in many forms such as tablets, capsules, softgels, gelcaps, liquids, or powders. The dietary supplement can also be used to promote energy to the dermal mitochondria, thus enhancing esthetic qualities of the skin.

The term "nutraceutical" as used herein denotes the usefulness in both the nutritional and pharmaceutical field of application. The nutraceutical compositions according to the present invention may be in any form that is suitable for administrating to the animal body including the human body, especially in any form that is conventional for oral administration, e.g. in solid form such as (additives/supplements for) food or feed, food or feed premix, tablets, pills, granules, dragées, capsules, and effervescent formulations such as powders and tablets, or in liquid form such as solutions, emulsions or suspensions as e.g. beverages, pastes and oily suspensions. Controlled (delayed) release formulations incorporating the hydroxytyrosol or olive juice extracts containing hydroxytyrosol according to the invention also form part of the invention. Furthermore, a multi-vitamin and mineral supplement may be added to the nutraceutical compositions of the present invention to obtain an adequate amount of an essential nutrient, which is missing in some diets. The multi-vitamin and mineral supplement may also be useful for disease prevention and protection against nutritional losses and deficiencies due to lifestyle patterns. The nutraceutical can further comprise usual additives, for example sweeteners, flavors, sugar, fat, emulgators, preservatives. The nutrition can also comprise other active components, such as (hydrolysed) proteins as described in for example WO 02/45524. Also anti-oxidants can be present in the nutrition, for example flavonoids, carotenoids, ubiquinones, rutin, lipoic acid, catalase, glutatione (GSH) and vitamins, such as for example C and E or their precursors.

In a further embodiment, a hydroxytyrosol containing composition is applied topically in order to enhance the mitochondrial biogenesis of dermal cells. The cosmetic or dermatological preparations according to the invention may be in the form of a suspension or dispersion in solvents or fatty substances, or alternatively in the form of an emulsion or micro emulsion (in particular of O/W or W/O type, O/W/O or W/O/W-type, wherein O stands for oil phase and wherein W stands for water phase), such as a cream, a paste, a lotion, a thickened lotion or a milk, a vesicular dispersion in the form of an ointment, a gel, a solid tube stick or an aerosol mousse, and may be provided in the form of a mousse, foam or a spray foams, sprays, sticks or aerosols or wipes. Examples of cosmetic or dermatological preparations are skin care preparations, in particular, body oils, body lotions, body gels, treatment creams, skin protection ointments, moisturizing gels, moisturizing sprays, revitalizing body sprays, after sun preparations or sunscreen formulations.

The cosmetic or dermatological preparations of the invention may further comprise the usual cosmetic respectively dermatological adjuvants and/or additives such as preservatives/ antioxidants, fatty substances/ oils, water, organic solvents, silicones, thickeners, softeners, emulsifiers, additional light screening agents, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, light stabilizers, insect repellants, skin tanning agents, skin whitening agents, antibacterial agents, preservatives active ingredients or any other ingredients usually formulated into cosmetics.

Generally between about 1 mg to about 500 mg of hydroxytyrosol in an olive extract is effective per serving. Preferably between 1 mg and 250 mg hydroxytyrosol is present in the olive extract, and even more preferably between about 1 mg and 100 mg in an olive extract is used

The daily dosage of hydroxytyrosol for humans (70 kg person) may be at least 0.1 mg. It may vary from 1 to 500 mg, preferably from 5 to 100 mg.

The preferred dose of hydroxytyrosol varies from 0.28 to 1.9 mg/kg metabolic body weight for mammals, whereby
"metabolic body weight" [in kg] = (body weight [in kg])^{0.75}
for mammals. Thatis means e.g. that for a human of 70 kg the preferred daily dose would vary between 6.77 and 45.98 mg, for a 20 kg dog the preferred daily dose would vary between 2.23 and 15.1 mg.

The following non-limiting Examples are presented to better illustrate the invention.

### EXAMPLES

### Example 1

Anti-rabbit PGC-1α and anti-rabbit PPAR-γ were purchased from Santa Cruz (California, USA); anti-α-tubulin from Sigma (St. Louis, MO, USA); Mito-Tracker Green FM, antioxidative complex I, II, III, and V from Invitrogen (Carlsbad, USA); SYBR® GREEN PCR Master Mix from ABI (Warrington, UK); BD Oxygen Biosensor System plate from BD Biosciences(California, USA); Hydroxytyrosol (DSM Nutritional Products); Mitochondrial D-loop and 18SRNA primers were synthesized by Bioasia Biotech (Shanghai, China), other reagents for cell culture were from Invitrogen (Carlsbad, USA).

### Cell culture and adipocyte differentiation

Murine 3T3-L1 pre-adipocytes (American Type Culture Collection) were cultured in Dulbecco's Modified Eagle's Medium (DMEM) supplemented with 10% fetal bovine serum and allowed to reach confluence. Differentiation of pre-adipocytes was initiated with 1 µM insulin, 0.25 µM dexamethasone and 0.5 mM 3-isobutyl-1-methylxanthine in DMEM, supplemented with 10% fetal bovine serum. After 48 h, the culture medium was replaced with DMEM supplemented with 10% fetal bovine serum and 1 µM insulin. The culture medium was changed every other day with DMEM containing 10% fetal bovine serum. Cells were used 9-10 days following differentiation induction when exhibiting 90% adipocyte phenotype.

### Determination of mitochondrial mass

Adipocytes were trypsinized and centrifuged at 1,000 rpm at 4°C for 5 min, resuspended in Kreb's Ringer solution buffered with HEPES and 0.1 % BSA, then incubated with 0.1 µM MitoTracker Green FM in DMEM for 30 min at 37°C. Cells were centrifuged at 1,000 rpm at 4°C for 5 min and resuspended in 400 µl of fresh Kreb's Ringer solution buffered with HEPES. To examine relative mitochondrial staining in the fractions, 20 × 10³ Mitotracker- labeled cells in 200 µl PBS from each fraction were loaded into a 96-well plate and relative fluorescence intensity was read (excitation 485 ± 25 nm; emission 538 ± 25 nm) using a fluorescence microplate spectrophotometer (Molecular probe). Results are expressed as fold increase of the fluorescence intensity over untreated control cells. Values are mean ± SE of the results from four independent experiments .

### Western blot analysis

After treatment, cells were washed twice with ice-cold PBS, lysed in sample buffer (62.5mM Tris-Cl pH 6.8, 2% SDS, 5mM DTT) at room temperature and vortexed. Cell lysates were then boiled for 5 minutes and cleared by centrifugation (13,000 rpm, 10 minutes at 4 °C). Protein concentration was determined using the Bio-Rad DC protein assay. The soluble lysates (10 µg per lane) were subjected to 10% SDS-PAGE, proteins were then transferred to nitrocellulose membranes and blocked with 5% non-fat milk/TBST for 1 h at room temperature. Membranes were incubated with primary antibodies directed against PPAR-γ (1:1000), PGC-1α (1:1000), α-tubulin (1:10 000), Complex I (1:2000), Complex II (1:2000), Complex III (1:2000) and Complex V (1:2000) in 5% milk/TBST at 4°C overnight. After washing membranes with TBST three times, membranes were incubated with horseradish peroxidase-conjugated secondary antibody for 1 h at room temperature. Western blots were developed using ECL (Roche Manheim, Germany) and quantified by scanning densitometry.

### Measurement of respiration in adipocytes

Oxygen consumption by intact cells was measured as an indication of mitochondrial respiration activity. The BD™ Oxygen Biosensor System (BD Biosciences, Franklin Lakes, NJ, USA) is an oxygen sensitive fluorescent compound (tris 1,7-diphenyl-1,10 phenanthroline ruthenium (II) chloride) embedded in a gas permeable and hydrophobic matrix permanently attached to the bottom of a multiwell plate. The concentration of oxygen in the vicinity of the dye is in equilibrium with that in the liquid media. Oxygen quenches the dye in a predictable concentration dependent manner. The amount of fluorescence correlates directly to the rate of oxygen consumption in the well, which in turn can relate to any sort of reaction that can be linked to oxygen consumption. The unique technology allows homogenous instantaneous detection of oxygen levels. After treatment, adipocytes were washed in KRH buffer plus 1% BSA. Cells from each condition were divided into aliquots in a BD Oxygen Biosensor System plate (BD Biosciences) in triplicate. Plates were sealed and "read" on a Fluorescence spectrometer (Molecular probes) at 1-minute intervals for 60 minutes at an excitation wavelength of 485 nm and emission wavelength of 630 nm. The number of cells contained in equal volumes was not statistically significantly between conditions (Wilson-Fritch et al., 2004 J Clin Invest 114:1281-1289).

### Measurement of mitochondrial DNA

Quantitative PCR was performed in Mx3000P Real-Time PCR system (Stratagene). Reactions were performed with 12.5 µl SYBR-Green Master Mix (ABI), 0.5 µl of each primer (10 µM), 100ng template (DNA) or no template (NTC), and RNase-free water was added to a final volume of 25 µl. The cycling conditions were as follows: 50°C for 2 min, initial denaturation at 95°C for 10 min, followed by 40 cycles of 95°C for 30 sec, 55°C for 1 min and 72°C for 30 sec. Each quantitative PCR was performed in triplicate. The following primers were used:
mitochondrial D-loop forward, 5'-AATCTACCATCCTCCGTG-3' (SEQ.ID.NO: 1)
reverse 5'-GACTAATGATTCTTCACCGT (SEQ. ID.NO:2)
18SRNA forward: 5'-CATTCGAACGTCTGCCCTATC-3' (SEQ.ID.NO:3) and
reverse: 5'-CCTGCTGCCTTCCTTGGA-3' (SEQ.ID.NO: 4)

The mouse 18S rRNA gene served as the endogenous reference gene. The melting curve was done to ensure specific amplification. The standard curve method was used for relative quantification. The ratio of mitochondrial D-loop to 18S rRNA was then calculated. Final results are presented as percentage of control.

### Assays for activities of mitochondrial complex I, II, and III

Adipocytes were cultured in 100 mm plates, washed in PBS, resuspended in an appropriate isotonic buffer (0.25 M sucrose, 5 mM Tris-HCl, pH 7.5, and 0.1 mM phenylmethylsulfonyl fluoride), and homogenized. Mitochondria were isolated by differential centrifugation of the cell homogenates. NADH-CoQ oxidoreductase (Complex I), succinate-CoQ oxidoreductase (complex II), CoQ-cytochrome c reductase (complex III) were assayed spectrometrically using the conventional assays (Picklo and Montine, 2001 Biochim Biophys Acta 1535: 145-152; Humphries, K. M., and Szweda, L. 1. 1998 Biochemistry 37:15835-15841), with minor modifications

### Assays for analysing expression of Cpt1a mRNA.

3T3-L1. Adipocytes were treated for 48 hrs with HT at 0.1, 1.0, 10, and 50 µmol/l, and total RNA was isolated. Cpt1a expression was analysed by RT-PCR using the conditions described in in Shen W, Liu K, Tian C, et al. R-alpha-Lipoic acid and acetyl-L: -carnitine complementarily promote mitochondrial biogenesis in murine 3T3-L1 adipocytes. Diabetologia 2008;51:165-174.

The cycle number at which the various transcripts were detectable was compared with that of 18S rRNA as an internal control.

### Statistical analysis

All qualitative data were representative of at least three independent experiments. Data are presented as means ± SE. Statistical significance was determined by using one-way ANOVA with Bonferroni's post hoc tests between the two groups. The criterion for significance was set at p<0.05.

### Results:

### Effect of Hydroxytyrosol on PGG1α protein level in adipocytes

As shown in Figure 1, hydroxytyrosol showed a bell-shape effect on increasing PGC-1α from 0.1 to 10.0 µM with a maximum protein expression at 1.0 µM (205±52%, p,0.05 vs.control).

### Effect of Hydroxytyrosol on complex I, II, III and V protein expression in adipocytes

Mitochondrial complexes was determined by western blot (Figure 2 A to D). An increase on mitochondrial electron transport complex I protein was observed with hydroxytyrosol treatment at 0.1 µM (131± 16%, p<0.05 vs.control), 1.0 µM (163 ± 31%, p<0.01 vs.control) and 10.0µM(138 ± 21%, p<0.05 vs.control) (Figure A). Complex II protein expression was also significantly increased with hydroxytyrosol at 0.1, 1.0 and 10.0µM (Figure B). Complex III and V protein expression was significantly increased with hydroxytyrosol at 0.1, 1.0 and 10.0µM (Figure C and D).

### Effects of Hydroxytyrosol on mitochondrial DNA

As the D-loop is known as the major site of transcription initiation on both the heavy and light strands of mtDNA, we examined *in vitro* whether hydroxytyrosol could increase mtDNA expression. As shown in Figure 3, the ratio of mt D-loop/18SRNA was significantly increased in adipocytes treated with hydroxytyrosol at 1.0µM.

### Effect of Hydroxytyrosol on oxygen consumption in adipocytes

To determine whether increased mitochondrial biogenesis is accompanied by changes in oxygen consumption, cells were treated with hydroxytyrosol at 0.1, 1.0,10 and 50µM. As shown in Figure 4, the basal rate of oxygen consumption was significantly increased in adipocytes treated with hydroxytyrosol at 1.0-10.0 µM.

### Effect of Hydroxytyrosol on activities of mitochondrial complex I, II, III, IV and V

Hydroxytyrosol showed significant increase in the activity of mitochondrial complex I in adipocytes cells at 1.0µM respectively relative to control (Figure 5A). The activity of mitochondrial complex II was significantly increased with hydroxytyrosol at 0.1, 1.0 and 10.0µM (Figure 5B). Hydroxytyrosol also showed significant increase in the activity of mitochondrial complex III, IV and V in adipocytes cells at 0.1 µM and 10µM (Figure 5C, 5D and 5E).

### Effect of Hydroxytyrosol on Cpt1 gene expression

CPT-1 is the gatekeeper of mitochondrial fatty acid oxidation because it regulates long-chain fatty acid transport across the mitochondrial membrane by converting acyl-CoA into acylcarnitine. HT showed dose-dependent increase on Cpt1 mRNA expressions significant increase at 1.0 µmol/l HT.

In summary hydroxytyrosol promotes mitochondrial activity and mitochondrial biogenesis leading to an enhancement of mitochondrial function and cellular defense system.

### Example 2

### Effect of Hydroxytyrosol on endurance

The purpose of the study was to determine the effect of hydroxytyrosol on maximal running performance on a treadmill.

In short, forty mice (C57BL/6NCrl; 8 wks old) were purchased from Charles River (Sulzfeld, Germany) and housed individually with free access to water and feed, with an alternating 12-hour light-dark cycle. The animals were fed a standard rodent diet (Ssniff R/M-H, Ext. n° V1536, Ssniff Ltd., Soest, Germany). After 2 weeks of adaptation to the diet the mice were randomized according to body weight into four experimental groups of 10 animals. The four groups were treated orally every morning for 3 weeks with either water (control group) or with an olive extract diluted to 0.2 ml with water and containing 50% hydroxytyrosol at doses of 50, 150, or 300 mg/kg body weight per day. There was no difference in feed consumption over the whole experiment. After 3 weeks of treatment the maximal running distance on a treadmill was measured. Statistical significance of the mean differences between dietary groups was tested by one-way analysis of variance (ANOVA). If significant differences were found, the Dunnett's test for multiple comparison was used to compare each group to the control group. P values less than 0.05 were considered significant. Hydroxytyrosol significantly increased the running distance to exhaustion in mice and so improved endurance in prolonged exercise.

FIGURE 7 shows the effect of Hydroxytyrosol, given as an olive extract containing 50% hydroxytyrosol at doses of 50, 150, or 300 mg/kg body weight per day by gavage, increases endurance by up to 50% in mice after 3 weeks of supplementation.

### Example 3

A 29 year old male fitness enthusiast drank a fitness water (such as Propel, Mizone or similar) comprising 50 mg hydroxytyrosol per 8 fl oz every day for 1 month before and during his regular endurance exercise. The hydroxytyrosol-containing fitness water helped increase his endurance.

### Example 4

A 22 year old female marathon runner drank a carbohydrate-rich sports beverage (such as Gatorade, Powerade, Lucozade or similar) containing 100 mg hydroxytyrosol per liter as the sole liquid source during her marathon run. This improved her endurance on the last 3 kilometers of the marathon, helping her to finish the race.

### Example 5

A supplement for body shaping / fat burning / improvement of body composition contains 100 mg hydroxytyrosol per daily dose.

## Claims

1. A method of maintaining or increasing mitochondrial biogenesis comprising administering an effective amount of hydroxytyrosol to a mammal.

2. A method according to claim 1 wherein the hydroxytyrosol is present in an olive juice extract.

3. A method according to Claim 1 or 2 wherein maintaining or increasing mitochondrial biogenesis results in maintaining or increasing cardiac energy, strength or endurance.

4. A method according to Claim 3 wherein increased cardiac strength results in increased blood circulation.

5. A method according to Claim 1 or 2 wherein maintaining or increasing mitochondrial biogenesis results in maintaining or increasing liver health, to assist the liver in maintaining healthy liver fat metabolism, or to maintain a healthy liver metabolism.

6. Use of hydroxytyrosol in the manufacture of a pharmaceutical, nutraceutical, or cosmetic composition for maintaining or increasing mitochondrial biogenesis.

7. Use according to Claim 6 wherein the hydroxytyrosol is present in an olive or olive juice extract

8. Use according to Claims 6 or 7 wherein maintaining or increasing mitochondrial biogenesis results in maintaining or increasing cardiac and/or skeletal muscle strength or endurance.

9. Use according to Claim 6 or 7 wherein maintaining or increasing mitochondrial biogenesis results in in maintaining or increasing liver health, assisting the liver in maintaining a healthy liver fat metabolism, or to maintain a healthy liver metabolism.

10. The use according to claim 1, wherein the composition is used for improving the body shape and/or for improving the muscle : fat ratio in mammals including humans.

11. A nutraceutical comprising hydroxytyrosol in an amount sufficient to maintain or increase mitochondrial biogenesis.

12. A composition comprising hydroxytyrosol to maintain or increase mitochondrial biogenesis.

13. A composition according to Claim 12 wherein maintained or increased mitochondrial biogensis results in a sustained energy boost.

14. A method of making a food composition which provides energy or alertness enhancement comprising adding hydroxytyrosol.

15. A method according to Claim 14 wherein the food composition contains a reduced caffeine, fat, and/or sugar content compared to a similar food item which does not contain hydroxytyrosol.


SEQUENCE LISTING

<110>  DSM IP Assets B.V.

<120>  HYDROXYTYROSOL BENEFITS MITOCHONDRIA

<130>  26764 WO

<150>  EP 08007492.5
<151>  2008-04-17

<160>  4

<170>  PatentIn version 3.3

<210>  1
<211>  18
<212>  DNA
<213>  Artificial

<220>
<223>  Primer

<400>  1
aatctaccat cctccgtg                                                    18


<210>  2
<211>  20
<212>  DNA
<213>  Artificial

<220>
<223>  Primer

<400>  2
gactaatgat tcttcaccgt                                                  20


<210>  3
<211>  21
<212>  DNA
<213>  Artificial

<220>
<223>  Primer

<400>  3
cattcgaacg tctgccctat c                                                21


<210>  4
<211>  18
<212>  DNA
<213>  Artificial

<220>
<223>  Primer

<400>  4
cctgctgcct tccttgga                                                    18

<110>  DSM IP Assets B.V.

<120>  HYDROXYTYROSOL BENEFITS MITOCHONDRIA

<130>  26764-EP-EPD

<140>  EP13197507.0
<141>  2013-12-16

<150>  EP 08007492.5
<151>  2008-04-17

<160>  4

<170>  PatentIn version 3.5

<210>  1
<211>  18
<212>  DNA
<213>  Artificial

<220>
<223>  Primer

<400>  1
aatctaccat cctccgtg                                                    18


<210>  2
<211>  20
<212>  DNA
<213>  Artificial

<220>
<223>  Primer

<400>  2
gactaatgat tcttcaccgt                                                  20


<210>  3
<211>  21
<212>  DNA
<213>  Artificial

<220>
<223>  Primer

<400>  3
cattcgaacg tctgccctat c                                                21


<210>  4
<211>  18
<212>  DNA
<213>  Artificial

<220>
<223>  Primer

<400>  4
cctgctgcct tccttgga                                                    18